(19) Europäisches Patentamt
European Patent Office
Office européen des brevets

(11) Publication number : 0 294 443 B1

# (12) EUROPEAN PATENT SPECIFICATION

(45) Date of publication of patent specification : **29.07.92 Bulletin 92/31**

(51) Int. Cl.$^5$ : **C07H 19/073,** C07H 19/09, A61K 31/70

(21) Application number : **88900484.2**

(22) Date of filing : **16.12.87**

(86) International application number : **PCT/SE87/00608**

(87) International publication number : **WO 88/04662 30.06.88 Gazette 88/14**

## (54) USE OF NUCLEOSIDES FOR THE MANUFACTURE OF MEDICAMENT FOR TREATMENT OF DISEASES CAUSED BY RETROVIRUS OR HEPATITIS B VIRUS.

The file contains technical information submitted after the application was filed and not included in this specification

(30) Priority : **19.12.86 SE 8605503**

(43) Date of publication of application : **14.12.88 Bulletin 88/50**

(45) Publication of the grant of the patent : **29.07.92 Bulletin 92/31**

(84) Designated Contracting States : **AT BE CH DE FR GB IT LI LU NL SE**

(56) References cited :
**EP-A- 0 196 185**
**WO-A-84/00759**
**WO-A-87/04929**
**DE-A- 1 620 185**
**DE-A- 2 915 254**
**DE-A- 3 036 131**
**DE-A- 3 045 375**
**DE-A- 3 045 375**
**US-A- 3 328 388**
**US-A- 3 328 388**
**US-A- 3 642 771**
**US-A- 4 247 544**
**US-A- 4 267 171**
**US-A- 4 344 937**
**US-A- 4 344 937**
**US-A-48 860 76**
**J. Carbohydrates-Nucleosides-Nucleotides, Vol., 5, No. 3, 1978, E. De Clercy et al, "Nucleoside analogs with selectiveantiviral activity", see pages 187-224**

(56) References cited :
**Methods and findings in experimental and clinical pharmacology, vol. 2, No. 5, 1980, E. De Clercq, "Antiviral and antitumoractivities of t-substituted 2l-deoxyuridines", see pages 253-267**
**Chemical Abstracts, Vol 104 (1986), abstract No. 180200n, Jpn. Kokai Tokkyo Koho. Jp 60.252.418**
**Journal of Medicinal Chemistry, Vol. 29, No. 9, 1986, E. De Clercq, "Chemotherapeutic Approaches to the Treatment of theQcquired Immune Deficiency Syndrome (AIDS)", see pages 1561-1569**

(73) Proprietor : **Medivir Aktiebolag**
**Lunastigen 7**
**S-141 44 Huddinge (SE)**

(72) Inventor : **DATEMA, Roelf**
**Krikongränd 8**
**S-151 44 Södertälje (SE)**
Inventor : **GOTTHAMMAR, Kristina, Birgitta**
**Björkholmsvägen 188**
**S-132 00 Saltsjö-Boo (SE)**
Inventor : **JOHANSSON, Karl, Nils, Gunnar**
**Bäverstigen 19**
**S-150 23 Enhörna (SE)**
Inventor : **KOVACS, Zsuzsanna, Maria, Ilona**
**Via Nazario Sauro, 118**
**I-17027 Pietra Ligure (IT)**
Inventor : **LINDBORG, Björn, Gunnar**
**Helgarögränd 14**
**S-125 42 Älvsjö (SE)**
Inventor : **STENING, Göran, Bertil**
**Varbovägen 10**
**S-151 50 Södertälje (SE)**
Inventor : **ÖBERG, Bo, Fredrik**
**Askvägen 27**
**S-752 52 Uppsala (SE)**

(74) Representative : **Larfeldt, Helene (SE) et al**
**Bergenstrahle & Lindvall AB Box 17704**
**S-118 93 Stockholm (SE)**

EP 0 294 443 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Field of the Invention

The present invention relates to the use of chemical compounds and physiologically acceptable salts thereof for the therapeutic and prophylactic control and treatment of the Acquired Immuno Deficiency Syndrome (AIDS), hepatitis B virus infections and retrovirus infections and method for such control and treatment in animal and man.

Background of the Invention

In the late seventies a new disease was reported, which subsequently was referred to as Acquired Immuno Deficiency Syndrome (AIDS). It is now generally accepted that a retrovirus referred to as Human T-cell Lymphotropic Virus (HTLV-III) or Lymphadenopathy Associated Virus (LAV) plays an essential role in the etiology of AIDS. This virus will herebelow be denoted by the new name HIV (Human Immunodeficiency Virus).

The full-blown AIDS is characterized by a profound immunodeficiency due to low numbers of a subset of lymphocyte-T-helper cells, which are one target for HIV infection. The profound immunodeficiency in the full-blown AIDS patients makes these patients highly susceptible to a variety of opportunistic infections of bacterial, fungal, protozoal or viral etiology. The etiological agents among viral opportunistic infections are often found in the herpes virus group, i.e., Herpes simplex virus (HSV), Varicella Zoster virus (VZV), Epstein-Barr virus (EBV) and, especially, cytomegalovirus (CMV). Other retroviruses affecting humans are HTLV-I and II and examples of retroviruses affecting animals are feline leukemia virus and equine infections anaemia virus.

Hepatitis B virus infections cause severe disease such as acute hepatitis, chronic hepatitis, fulminant hepatitis in a considerable number of persons. It is estimated that there are 200 million patients with chronic hepatitis B infection in the world. A considerable number of the chronic cases progress to liver cirrosis and liver tumours. In some cases the hepatitis infections also take a rapid and severe course as in fulminant B hepatitis with about 90 % mortality. At present there is no known effective treatment against hepatitis B infections.

Prior Art

Several nucleosides of the formula I below are known compounds. Some of the compounds are described for instance in J. Med. Chem. 26(9), 1252-7, J. Org. Chem. 43(14), 2870-6, DD 114949, US 4247544 and GB 1601020.

Structurally similar compounds reported to have antiviral activity towards DNA viruses are known also from the following references. Methods and findings in experimental and clinical pharmacology, Vol. 2, No. 5, 1980, pages 253-267;
US-A-4 267 171; DE-A-3 036 131; US-A-4 386 076; US-A-3 642 771; DE-A-1 620 185; US-A-3 328 388; DE-A-3 045 375; US-A-4 344 937; WO-A-84/00769.

In J.Carbohydrates-Nucleosides-Nucleotides, Vol. 5, No. 3, 1978, pages 187-224, 5-bromo- and 5-iododeoxy-uridine is reported to have activity against Rous sarcoma virus.

Disclosure of the Invention

It has been found according to the present invention that the compounds of the formula

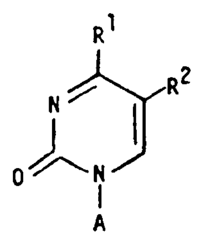

I

wherein
A is β-2'-deoxy-D-ribofuranosyl or β-D-arabinofuranosyl;
$R^1$ is hydroxy or amino;
$R^2$ is cycloalkyl or alkyl-substituted cycloalkyl containing 3-5 carbon atoms; a saturated or unsaturated, straight

or branched aliphatic group containing 2-5 carbon atoms which may be unsubstituted or substituted with halogen, a saturated or unsaturated straight or branched aliphatic group containing 1-5 carbon atoms substituted with hydroxy, mercapto, trifluoroalkyl or difluoroalkyl containing 1-3 carbon atoms, phenoxy or alkoxy containing 1-3 carbon atoms; phenyl or phenylalkyl containing 1-3 carbon atoms in the alkyl part, or a physiologically acceptable salt thereof,
present a new possibility to block the multiplication of retrovirus and hepatitis B virus, respectively, by use a nucleoside analogue of said formula. Accordingly, the nucleoside analogues of said formula and physiologically acceptable salts thereof have unobvious and beneficial properties as prophylactic and/or therapeutic agents in the control or treatment of retrovirus and hepatitis B virus infections, respectively. Said nucleosides are especially interesting as agents capable of inhibiting the activity of the HIV in man.

All retroviruses, including HIV, require an enzyme called reverse transcriptase in their natural cycle of replication.

Hepatitis B virus (HBV) is a DNA virus with a unique circular double-stranded DNA genome which is partly single-stranded. It contains a specific DNA polymerase required for viral replication. This DNA polymerase also acts as a reverse transcriptase during the replication of HBV DNA via an RNA intermediate.

The compounds of the invention can be transformed by cells or enzymes to triphosphates which inhibit the activity of reverse transcriptase of retrovirus and the activity of DNA polymerase of hepatitis B virus.

Individual compounds within the formula I may be novel.

The known compounds as well as the novel compounds of formula I are prepared in known manner as is described in the literature.

The uracil moiety of the 5-substituted deoxyuridine compounds and of the 5-substituted β-D-arabinofuranosyluracil compounds may be converted to a cytosine moiety of the corresponding deoxycytidine and β-D-arabinofuranosylcytosine analogues, by conventional methods, the principles of which have been described for example by W.L. Sung (J. Chem. Soc. Chem. Commun. 1981, p. 1089 and J. Organic Chemistry 1982, volume 47, pages 3623-3628) and by P. Herdewijn et al. (J. Medicinal Chemistry 1985, volume 28, pages 550-555).

The following nucleoside compounds constitute part of the invention as prophylactic and therapeutic agents in control or treatment of retrovirus, especially HIV, or hepatitis B virus infections:

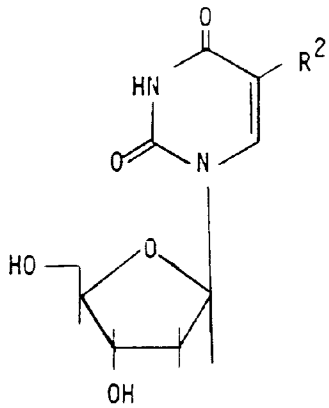

IA 5-$R^2$-2'-deoxyuridine

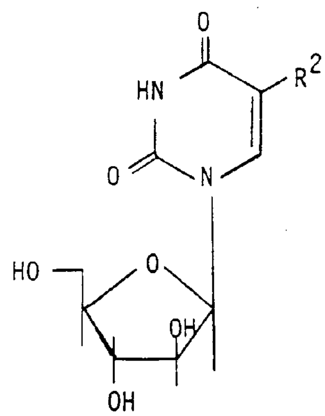

IB 1-(β-D-arabinofuranosyl)-5-$R^2$-uracil

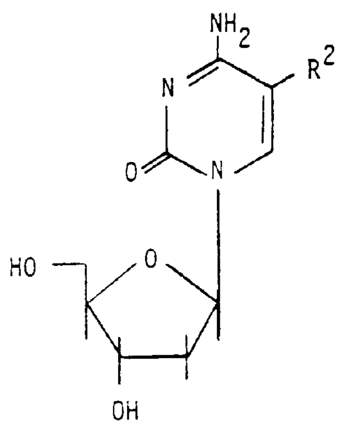

IC 5-$R^2$-2'-deoxycytidine

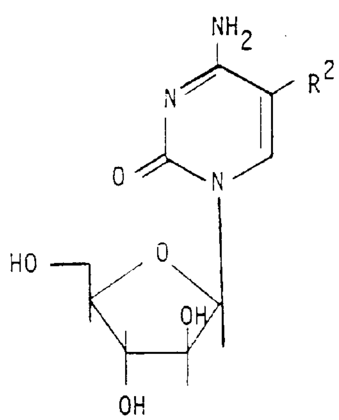

ID 1-(β-D-arabinofuranosyl)-5-$R^2$-cytosine

wherein $R^2$ may have the following meanings

alkyl: $-CH_2CH_3$, $-CH_2CH_2CH_3$, $-CH_2CH_2CH_2CH_3$, 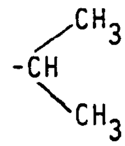 , 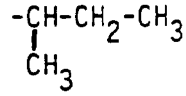, 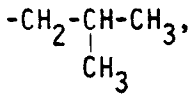, 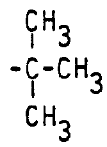

cycloalkyl: 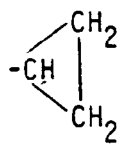 , 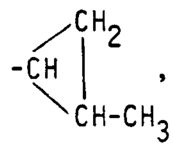 , 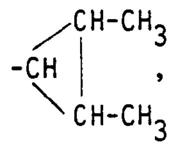 , 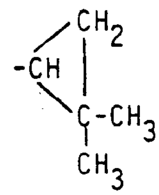 ,

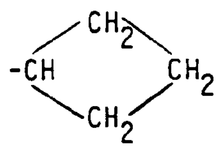

phenyl, phenylalkyl : 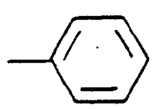 , 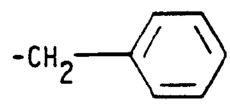 ;

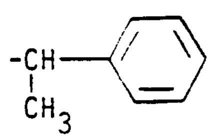 , 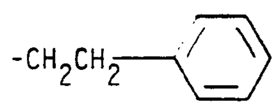

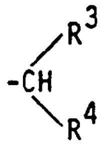

| $R^3$ | $R^4$ |
|---|---|
| H | OH |
| $CH_3$ | OH |
| $CH_2CH_3$ | OH |
| $CH_2CH_2CH_3$ | OH |
| H | $CH_2OH$ |
| $CH_3$ | $CH_2OH$ |
| $CH_2CH_3$ | $CH_2OH$ |
| H | SH |
| $CH_3$ | SH |
| $CH_2CH_3$ | SH |
| $CH_2CH_2CH_3$ | SH |
| H | $CH_2CH_2OH$ |
| $CH_3$ | $CH_2CH_2OH$ |
| H | $OCH_3$ |
| $CH_3$ | $OCH_3$ |
| H | 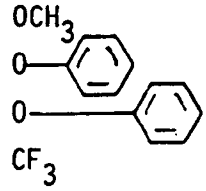 |
| $CH_3$ | |
| H | $CF_3$ |
| $CH_3$ | $CF_3$ |
| $CH_2CH_3$ | $CF_3$ |
| H | $CF_2CH_3$ |
| $CH_3$ | $CF_2CH_3$ |
| $CH_3$ | F |
| $CH_3$ | Cl |
| $CH_3$ | Br |
| $CH_3$ | I |
| $CH_2CH_3$ | F |
| $CH_2CH_3$ | Cl |
| $CH_2CH_3$ | Br |
| $CH_2CH_3$ | I |

| | |
|---|---|
| $-CH{=}CH{-}R^5$ | $R^5$: H, $CH_3$, $CH_2CH_3$, $CF_3$, $OCH_3$, $CH_2OH$, F, Cl |
| $-C(R^5){=}CH{-}R^5$ | $R^5$ may be the same or different and chosen among the following groups: H, $CH_3$, $CH_2CH_3$, $CF_3$, $OCH_3$, $CH_2OH$, F, Cl |
| $-C{\equiv}C{-}R^5$ | $R^5$: H, $CH_3$, $CH_2CH_3$, $CF_3$, $OCH_3$, $CH_2OH$, F, Cl |
| $-CH{=}C{=}CH_2$ | |

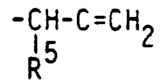

| $R^5$ |
|---|
| H |
| $CH_3$ |
| $CH_2CH_3$ |
| $CF_3$ |
| $OCH_3$ |
| $CH_2OH$ |
| F |
| Cl |

Nucleosides containing uracil as base are preferred, as agents for use in control or treatment of retrovirus, especially HIV and hepatitis B virus infections in animal and man. The following nucleosides are the most preferred compounds: 5-cyclopropyl-2′-deoxyuridine, 5-ethyl-2′-deoxyuridine, 5-hydroxymethyl-2′-deoxyuridine, 5-(1-propenyl)-2′-deoxyuridine, 1-(β-D-arabinofuranosyl)-5-(1-propenyl)-uracil, 5-isopropenyl-2′-deoxyuridine, or a physiologically acceptable salt thereof.

In clinical practice the nucleosides of the formula I will normally be administered orally, by injection or by infusion in the form of a pharmaceutical preparation comprising the active ingredient in the form of the original compound or optionally in the form of a pharmaceutically acceptable salt thereof, in association with a pharmaceutically acceptable carrier which may be a solid, semi-solid or liquid diluent or an ingestible capsule. The compound may also be used without carrier material. As examples of pharmaceutical preparations may be mentioned tablets, dragées, capsules, granulates, suspensions, elixirs, syrups, solutions etc. Usually the active substance will comprise between 0.05 and 20 % for preparations intended for injection and between 10 and 90 % for preparations intended for oral administration.

In the treatment of patients suffering from retrovirus, especially HIV, or hepatitis B virus infections, it will be preferred to administer the compounds orally, parenterally or intravenously. The dosage at which the active ingredients are administered may vary within a wide range and will depend on various factors such as the severity of the infection, the age of patient etc., and may have to be individually adjusted. As a possible range for the amount of the compounds of the invention or a physiologically acceptable salt thereof to be administered per day may be mentioned from about 10 mg to about 10 000 mg, preferentially 100-500 mg for intravenous administration and preferentially 100-1000 mg for oral administration.

Salts

The physiologically acceptable salts are suitable acid addition salts, preferably derived from non-toxic acids. Such acid addition salts include, for example, those derived from hydrochloric acid, hydroiodic acid, sulphuric acid, p-toluenesulphonic acid, methanesulphonic acid, maleic acid, lactic acid, citric acid, tartaric acid, succinic acid, oxalic acid, p-chlorobenzenesulphonic acid, phosphoric acid, acetic acid, gluconic acid, pantothenic acid and lactobionic acid.

Experimental test

Test I. Effect of compounds of the formula I of HIV in H9 cells

Materials and methods: HIV infection of H9 cells

H9 cells, $10^5$ cells per well on a 24 well plate, suspended in 2 ml RPMI-medium containing 10% fetal calf serum, 100 μg/ml penicillin, 10 μg/ml streptomycin sulfate and 2 μg/ml polybrene are exposed to HIV (HTLV-$III_B$) and different concentrations of the inhibiting compounds. The plates are incubated at 37°C in 5% $CO_2$ for 6-7 days. The contents in each well is then homogenized with a pipette and transferred to a centrifuge tube. After centrifugation for 10 min at 1500 rpm the supernatent is removed and the cell pellet is analyzed by fixing in methanol on glass plates. Human HIV positive serum diluted 1:80 or 1:160 is added and incubated for 30 min

at 37°. The plate is then washed with phosphate-buffered saline (PBS) containing $Ca^{2+}$ and $Mg^{2+}$. Sheep anti-human conjugate (FITC) is added and after a new incubation the plate is again washed with PBS. Contrast staining is done with Evans blue and after drying the frequency of HIV antigen containing cells is determined in a microscope. The test result is shown in Table 1.

Table 1. Concentration (µM) for 50% inhibition ($IC_{50}$) of human immunodeficiency virus multiplication in cell culture

| Compounds | $IC_{50}$ (µM) |
|---|---|
| 5-cyclopropyl-2'-deoxyuridine | < 1 |
| 5-ethyl-2'-deoxyuridine | 1 |
| 5-hydroxymethyl-2'-deoxyuridine | < 1 |
| 5-(1-propenyl)-2'-deoxyuridine | < 1 |
| 1-(β-D-arabinofuranosyl)-5-(1-propenyl)-uracil | < 1 |

## Claims

1. Use of a compound of the formula

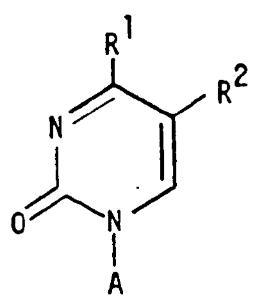

I

wherein A is

β-2′-deoxy-D-ribofuranosyl or

β-D-arabinofuranosyl;

$R^1$ is hydroxy or amino;

$R^2$ is cycloalkyl or alkyl-substituted cycloalkyl containing 3-5 carbon atoms; a saturated or unsaturated, straight or branched aliphatic group containing 2-5 carbon atoms which may be unsubstituted or substituted with halogen, a saturated or unsaturated straight or branched aliphatic group containing 1-5 carbon atoms substituted with hydroxy, mercapto, trifluoroalkyl or difluoroalkyl containing 1-3 carbon atoms, phenoxy or alkoxy containing 1-3 carbon atoms; phenyl or phenylalkyl containing 1-3 carbon atoms in the alkyl part, or a physiologically acceptable salt thereof,

for manufacture of a medicament for therapeutic or prophylactic control or treatment of retrovirus, especially HIV, or hepatitis B virus infections in animal and man.

2. Use according to claim 1 of a compound of formula I wherein $R^1$ is hydroxy, or a physiologically acceptable salt thereof.

3. Use according to claims 1-2 of a compound of formula I wherein $R^2$ is cycloalkyl, hydroxyalkyl, a saturated or unsaturated aliphatic group containing 2-3 carbon atoms, or a physiologically acceptable salt thereof.

4. Use of 5-cyclopropyl-2′-deoxyuridine, 5-ethyl-2′-deoxyuridine, 5-hydroxymethyl-2′-deoxyuridine, 5-(1-propenyl)-2′-deoxyuridine, 1-(β-D-arabinofuranosyl)-5-(1-propenyl)-uracil, 5-isopropenyl-2′-deoxyuridine, or a

physiologically acceptable salt thereof for manufacture of a medicament for therapeutic or prophylactic control or treatment of retrovirus, especially HIV, or hepatitis B virus infections in animal and man.

5. Use of 5-cyclopropyl-2′-deoxyuridine, 5-ethyl-2′-deoxyuridine, 5-hydroxymethyl-2′-deoxyuridine, 5-(1-propenyl)-2′-deoxyuridine, 1-(β-D-arabinofuranosyl)-5-(1-propenyl)-uracil, 5-isopropenyl-2′-deoxyuridine, or a physiologically acceptable salt thereof for manufacture of a medicament for therapeutic or prophylactic control or treatment of acquired immuno deficiency syndrome (AIDS) or other symptoms caused by HIV in man.

## Patentansprüche

1. Verwendung einer Verbindung der Formel

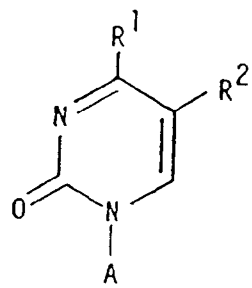

worin A

β-2′-Deoxy-D-ribofuranosyl oder
β-D-Arabinofuranosyl;

$R^1$ Hydroxy oder Amino;

$R^2$ ein Cycloalkyl oder alkyl-substituiertes Cylcoalkyl mit 3 bis 5 Kohlenstoffatomen; eine gesättigte oder ungesättigte, geradkettige oder verzweigtkettige aliphatische Gruppe mit 2 bis 5 Kohlenstoffatomen, die unsubstituiert oder mit Halogen substituiert sein kann; eine gesättigte oder ungesättigte, geradkettige oder verzweigtkettige aliphatische Gruppe mit 1 bis 5 Kohlenstoffatomen, substituiert mit Hydroxy, Mercapto; Trifluoralkyl oder Difluoralkyl mit 1 bis 3 Kohlenstoffatomen; Phenoxy oder Alkoxy mit 1 bis 3 Kohlenstoffatomen; Phenyl oder Phenylalkyl mit 1 bis 3 Kohlenstoffatomen im Alkylrest, oder ein physiologisch akzeptables Salz derselben ist,

für die Herstellung eines Medikamentes zur therapeutischen oder prophylaktischen Kontrolle oder Behandlung von retroviralen, insbesondere HIV oder Hepatitis- B-Virusinfektionen in Tier und Mensch.

2. Verwendung nach Anspruch 1 einer Verbindung gemäß Formel I, wobei $R^1$ Hydroxy ist oder ein physiologisch akzeptables Salz derselben.

3. Verwendung nach einem der Ansprüche 1 bis 2 einer Verbindung gemäß Formel I, wobei $R^2$ Cycloalkyl, Hydroxyalkyl, eine gesättigte oder ungesättigte aliphatische Gruppe mit 2 bis 3 Kohlenstoffatomen ist oder ein physiologisch akzeptables Salz derselben ist.

4. Verwendung von 5-Cyclopropyl-2′-deoxyuridin, 5-Ethyl-2′-deoxyuridin, 5-Hydroxymethyl-2′-deoxyuridin, 5-(1-Propenyl)-2′-deoxyuridin, 1-(β-D-Arabinofuranosyl)-5-(1-propenyl)-uracil, 5-Isopropenyl-2′-deoxyuridin oder ein physiologisch akzeptables Salz derselben für die Herstellung eines Medikaments zur therapeutischen oder prophylaktischen Kontrolle oder Behandlung von retroviralen, insbesondere HIV oder Hepatitis-B-Virusinfektionen in Tier und Mensch.

5. Verwendung von 5-Cyclopropyl-2′-deoxyuridin, 5-Ethyl-2′--deoxyuridin, 5-Hydroxymethyl-2′-deoxyuridin, 5-(1-Propenyl)-2′-deoxyuridin, 1-(β-D-Arabinofuranosyl)-5-(1-propenyl)-uracil, 5-Isopropenyl-2′-deoxyuridin oder eines physiologisch akzeptablen Salzes derselben für die Herstellung eines Medikaments zur therapeutischen oder prophylaktischen Kontrolle oder Behandlung des Erworbenen Immunschwächesyndroms (AIDS) oder anderer Symptome, die von HIV beim Menschen verursacht werden.

## Revendications

1. Utilisation d'un composé de formule :

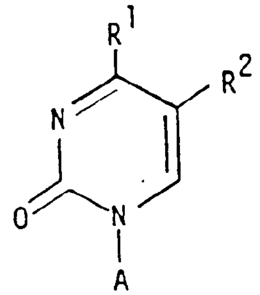

I

dans laquelle : A est un groupe

β-2′-désoxy-D-ribofuranosyle ou

β-D-arabinofuranosyle;

$R^1$ est un groupe hydroxy ou amino;

$R^2$ est un groupe cycloalkyle ou cycloalkyle à substituant alkyle contenant 3 à 5 atomes de carbone; un groupe aliphatique saturé ou insaturé à chaîne droite ou ramifiée, contenant 2 à 5 atomes de carbone qui peut être non substitué ou substitué avec un atome d'halogène, un groupe aliphatique saturé ou insaturé à chaîne droite ou ramifiée, contenant 1 à 5 atomes de carbone substitué avec un groupe hydroxy, mercapto, trifluoroalkyle ou difluoroalkyle contenant 1 à 3 atomes de carbone, phénoxy ou alcoxy contenant 1 à 3 atomes de carbone; un groupe phényle ou phénylalkyle contenant 1 à 3 atomes de carbone dans la partie alkyle, ou d'un sel de celui-ci acceptable du point de vue physiologique,

pour la fabrication d'un médicament pour le contrôle ou le traitement thérapeutique ou prophylactique d'infections provoquées par un rétrovirus, en particulier le HIV, ou le virus de l'hépatite B chez l'animal et l'homme.

2. Utilisation suivant la revendication 1 d'un composé de formule I dans laquelle $R^1$ est un groupe hydroxy, ou d'un sel de celui-ci acceptable du point de vue physiologique.

3. Utilisation suivant les revendications 1 et 2 d'un composé de formule I dans laquelle $R^2$ est un groupe cycloalkyle, hydroxyalkyle, un groupe aliphatique saturé ou insaturé contenant 2 à 3 atomes de carbone, ou d'un sel de celui-ci acceptable du point de vue physiologique.

4. Utilisation de la 5-cyclopropyl-2′-désoxyuridine, la 5-éthyl-2′-désoxyuridine, la 5-hydroxyméthyl-2′-désoxyuridine, la 5-(1-propényl)-2′-désoxyuridine, du 1-(β-D-arabinofuranosyl)-5-(1-propényl)-uracile, la 5-isopropényl-2′-désoxyuridine, ou d'un sel de ceux-ci acceptable du point de vue physiologique pour la fabrication d'un médicament pour le contrôle ou le traitement thérapeutique ou prophylactique d'infections provoquées par un rétrovirus, en particulier le HIV, ou le virus de l'hépatite B chez l'animal et l'homme.

5. Utilisation de la 5-cyclopropyl-2′-désoxyuridine, la 5-éthyl-2′-désoxyuridine, la 5-hydroxyméthyl-2′-désoxyuridine, la 5-(1-propényl)-2′-désoxyuridine, du 1-(β-D-arabinofuranosyl)-5-(1-propényl)-uracile, la 5-isopropényl-2′-désoxyuridine, ou d'un sel de ceux-ci acceptable du point de vue physiologique pour la fabrication d'un médicament pour le contrôle ou le traitement thérapeutique ou prophylactique du syndrome d'immunodéficience acquise (SIDA) ou d'autres symptômes provoqués par le HIV chez l'homme.